# EUROPEAN PATENT SPECIFICATION

(11) **EP 2 663 352 B1**
(45) Date of publication and mention of the grant of the patent: **27.04.2016**
(21) Application number: 11855695.0
(22) Date of filing: 11.01.2011
(51) Int. Cl.: A61M 5/50, A61M 5/34, A61M 5/32

(54) **AUTO-DISABLE SAFETY SYRINGE**
SELBSTDEAKTIVIERENDE SICHERHEITSSPRITZE
SERINGUE DE SÉCURITÉ AUTOBLOQUANTE

(43) Date of publication of application: 20.11.2013
(73) Proprietor: Abu Dhabi National Industrial Projects Co., Abu Dhabi (AE)
(72) Inventor: HAWASHEEN, Amer, W., Y., Abu Dhabi (AE)
(74) Representative: Tansini, Elio Fabrizio
(86) International application number: PCT/IB2011/000025
(87) International publication number: WO 2012/095679

(56) References cited:
- EP-A1- 2 153 856
- EP-A1- 2 153 856
- WO-A1-92/04064
- WO-A1-92/11883
- WO-A1-96/32977
- WO-A1-98/53867
- WO-A1-98/53867
- WO-A1-03/045476
- WO-A1-2008/111931
- WO-A1-2009/140801
- WO-A1-2009/140801
- US-A- 5 613 951
- US-A1- 2002 161 340
- US-A1- 2003 212 366
- US-A1- 2003 212 366
- US-A1- 2004 230 163
- US-A1- 2005 277 880
- US-A1- 2006 235 074
- US-A1- 2007 078 402
- US-A1- 2009 234 284

## Description

### Field of the Invention

The invention pertains to single use disposable syringes having a retractable needle.

### Background of the Invention

Conventional disposable hypodermic syringes present two distinct health hazards. One is the accidental scratching or puncturing by the needle of the skin of the person administering an injection after withdrawal from the patient or during disposal. The other is the intentional re-use of the syringe, even though intended for a single use, such as reuse by drug addicts. Both hazards can result in the spread of infectious diseases where the syringe is contaminated as a result of use.

Various designs of syringes have been made to address one or the other of these concerns. Mechanisms have been designed for the retraction of the needle into the syringe barrel after use, so that the barrel acts as a protective sheath. Examples in the patent literature include Lake, WO 95/03845 A1; Jenson, US 5,380,285; and Smith et al., US 2004/0087907 A1. Mechanisms which disable the syringe by preventing refilling after a single injection have also been designed, for example Meyer et al., US 5,613,951; Butler et al., US 4,941,879; and Richardson et al., WO 92/04064 A1. Goossens et al., WO 2005/023344 A1 discloses a design in which a closure element blocks the fluid passageway, creating a vacuum in the syringe barrel when the plunger is retracted, withdrawing the needle into the barrel.

Further examples of known syringes are disclosed in documents EP 2153856, US 2003/212366, WO 98/53867 and WO 2009/140801.

The present invention is directed to improvements in the art of single use safety syringes to reduce the foregoing hazards.

### Summary of the Invention

The invention is defined in claim 1.

The invention provides a single use syringe in which the syringe may not be refilled after one use and in which the needle may be withdrawn into the syringe barrel. The syringe has a barrel and a plunger, a needle connector at the distal end of the barrel having a portion within the barrel bore and a portion extending from an opening in the distal end of the barrel, the needle connector being in sealing, releasable engagement with the barrel wall, and a needle hub affixed to the needle connector and having a hypodermic needle fixed thereto. The needle connector and the needle hub form an assembly, the assembly having a cavity therein. The needle and the barrel bore are in fluid communication through the cavity. An elastically-deformable sealing ring having an opening and a moveable sealing member are positioned in the cavity. The sealing member is moveable from a first position in which its head is on the proximal side of the ring to a second position in which the head is on the distal side of the ring; the ring being configured to stop movement of the sealing member from the second position to the first position and to form a seal with the sealing member, when the sealing member is in the second position, against a flow of fluid in the proximal direction. This mechanism provides a one-way valve in the syringe. There are locking means for locking together the plunger and the needle connector such that the needle is retractable into the barrel bore, when the plunger and the needle connector are locked together, by movement of the plunger in the proximal direction.

The one-way valve operates after some volume of fluid is expelled during an injection but it does not require that the plunger be in a fully inserted position within the barrel, i.e. that the barrel be emptied. It thus prevents misuse by refilling even where the barrel has been only partly emptied. At the same time, the syringe provides a mechanism for mechanically locking the plunger to the needle assembly, providing for secure retraction of the needle into the barrel after use.

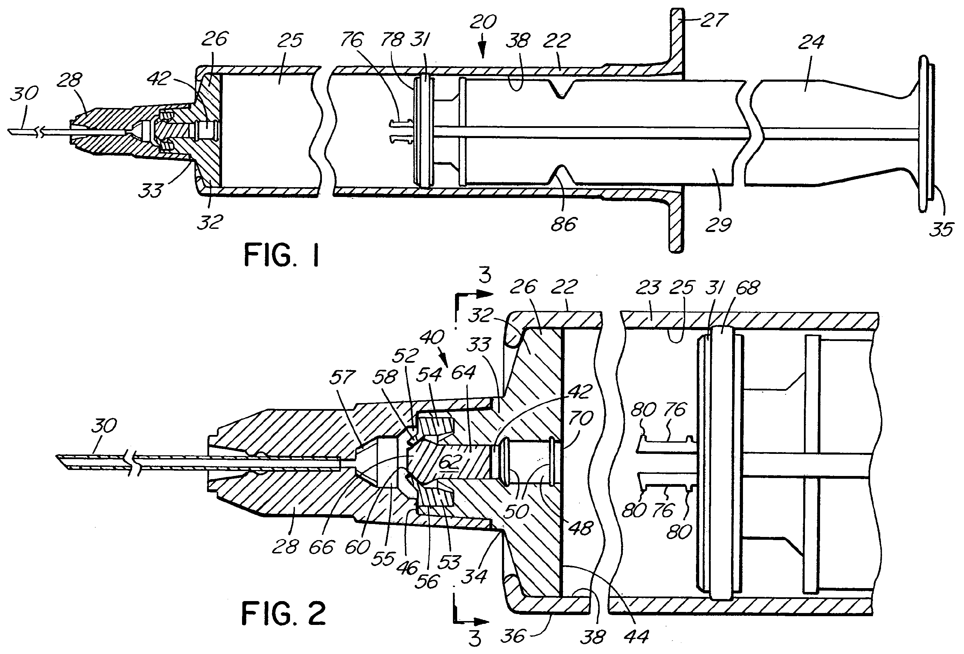

In the description of the syringe herein, "distal" indicates the needle end of the syringe (i.e. the left side in Figure 1) and "proximal" indicates the opposite, plunger end (i.e. the right side in Figure 1).

Further aspects of the invention and features of specific embodiments are described below.

### Brief Description of the Drawings

In drawings in which corresponding and like parts are identified by the same reference characters:
Figure 1 is a side elevation view, partly cutaway, of a syringe not according to the invention.
Figure 2 is a longitudinal cross-sectional view of the distal portion of the syringe of Figure 1, shown ready for injection.
Figure 3 is a cross-sectional view on the line 3-3 of Figure 2.
Figure 4 is a longitudinal cross-sectional view of a portion of the distal portion of the syringe, shown after the injection has commenced, with the moveable sealing member in the position which prevents refilling.
Figure 5 is a longitudinal cross-sectional view of the syringe of Figure 1, shown at the end of an injection stroke, with the plunger and needle connector locked together.
Figure 6 is a longitudinal cross-sectional view of the syringe of Figure 1, shown with the needle withdrawn into the syringe barrel after use.
Figure 6A is a detailed view of a portion of Figure 6.
Figure 7 is a longitudinal cross-sectional view of the distal portion of a syringe not according to the invention, shown after actuation of the moveable sealing member and before the locking of the plunger to the needle connector.
Figure 8 is a view similar to Figure 7, shown after locking of the plunger to the needle connector.
Figure 9 is an isometric view of the distal portion of the plunger of the syringe of Figure 7.
Figure 10 is a longitudinal cross-sectional view of the distal portion of a syringe in accordance with the invention, shown after actuation of the moveable sealing member and with the plunger locked to the needle connector.
Figure 11 is an isometric view of the distal portion of the plunger of the syringe of Figure 10.
Figure 12 is an isometric view of the needle connector of the syringe of Figure 10.

### Detailed Description

Referring first to Figures 1 to 6, which show a first embodiment of the safety syringe, the single use hypodermic syringe **20** includes a barrel **22,** a plunger **24,** a needle connector **26** and a needle hub **28** with a hypodermic needle **30** affixed to it. The barrel **22** has a barrel wall **23** defining a bore **25.** The barrel has a radially outward outwardly-extending finger flange **27** at its proximal end. The plunger **24** includes a plunger shaft or rod **29,** a piston **31** at its distal end and a thumb rest **35** at its proximal end. The plunger shaft is cross-vaned in structure. An O-ring **68** on the piston **31** forms a fluid seal against the inner surface of the barrel wall **23.**

The needle connector **26** is located at the distal end of the barrel **22** and has a first portion **32** lying within the bore and a second portion **33** which extends out of the opening **34** in the distal end of the barrel. The radially outer surface **36** of the first portion **33** of the needle connector sealingly engages against the inner surface **38** of the barrel wall, such that fluid in the barrel bore **25** cannot escape between them. The radially outer surface **36** of the needle connector and the inner surface **38** of the barrel wall are, at their point of engagement, contoured to create a releasable snap-fit of the needle connector within the barrel. The snap-fit mechanism is such that the needle connector can be withdrawn into the barrel for purposes of sheathing the needle, as described below, but it is sufficiently strong to prevent movement of the needle connector during the manufacturing process, shipping, or during the filling and injection steps that occur during use of the syringe.

The needle connector **26** has a channel **42** which extends from the proximal side **44** of the needle connector to its distal side **46.** The channel **42** has an enlarged portion **48** adjacent to the proximal side **44,** with two circumferential grooves **50,** for engaging the plunger latching members, as described below.

The needle connector **26** and needle hub **28** attach securely together by means of circumferentially-disposed ribs **39** on the needle connector which interfit tightly with mating grooves **41** on the needle hub. Thus, when the needle connector is withdrawn into the barrel, the needle hub and needle are likewise retracted. The needle connector and the needle hub, being affixed together, form an assembly **40** having a cavity **52** therein. Part of the cavity comprises the channel **42** extending between the proximal and distal faces of the needle connector. Another part of the cavity **52** is the portion **53,** within the needle connector, configured to hold the annular base portion **54** of the sealing ring **56.** Another part of the cavity **52** is the portion **55** within the needle hub, between the distal end of the needle connector and the proximal end of the needle.

The sealing ring **56** has an elastically-deformable, inwardly-extending sealing portion **58,** extending from the annular base portion **54,** having an opening **60** therein. A moveable sealing member **62** has a shaft portion **64** and a head portion **66.** The shaft portion is positioned in the channel **42** of the needle connector. The head **66** of the moveable sealing member has a diameter larger than the diameter of the opening **60** of the sealing ring **56.** The sealing portion **58** of the sealing ring is distally inwardly inclined and is elastically deformable such that the head **66** of the moveable sealing member can pass through the opening **60** in the distal direction, and pass into the portion **55** of the cavity **52** in the needle hub under the pressure of liquid caused when the plunger in the syringe is moved in the distal direction.

The channel **42** in the needle connector **26** has four longitudinally-extending grooves **43** along the sides of the part of the channel **42** that accommodates the moveable sealing member **62.** The grooves permit the flow of liquid along the channel **42** past the sealing member **62.** Thus, liquid can flow through the channel **42** for filling the syringe and for injection. In the cavity portion **55** in the needle hub, channels **57** are provided in the cavity walls, adjacent to the distal end of the cavity, to permit the flow of liquid past the head **66** of the sealing member when the head **66** is in the cavity portion **55.**

Two locking pins **76** extend distally from the distal face **78** of the piston, proximate to its center, and aligned with the opening **70** of the channel **42** at the proximal side of the needle connector. The locking pins **76** are sized and positioned such that, when the distal face **78** of the piston is brought into contact with the proximal side **44** of the needle connector, the locking pins **76** are within the enlarged portion **48** of the channel **42** and the two ribs **80** on the radially outer surfaces of the locking pins are fitted in respective grooves **50** of the channel **42,** in a snap-fit engagement therewith.

The barrel **22** is designed to prevent removal of the needle after use. A circumferential groove **82** and lip **84** are provided in the inner surface of the barrel wall **23** close to its proximal end, to engage the radially outer periphery **36** of the needle connector and the O-ring respectively, when the plunger is retracted after use. A frangible or weakened portion **86** of the plunger shaft **29** is provided to permit the shaft to be broken off by a user when the needle is retracted to the locked position within the barrel.

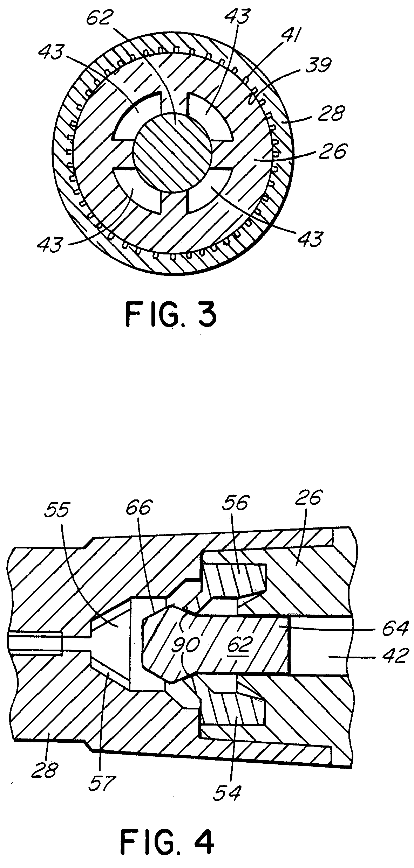

For initial filling of the syringe, the sealing member **62** is on the proximal side of the sealing ring **56,** as shown in Figure 2. Liquid can then be drawn into the barrel bore by movement of the plunger in the proximal direction, the liquid passing through the needle into the cavity portion **55,** through the opening **60** in the sealing ring **56,** through the channel **42** in the needle connector **26** and into the barrel bore. When the plunger is then pressed in the distal direction to inject the liquid, the liquid flows from the barrel bore through the channel **42,** pressing against the proximal side of the sealing portion **58** of the ring **56** and against the proximal end of the moveable sealing member **62.** This pressure causes the opening **60** of the sealing ring **56** to enlarge and permit the head **66** of the moveable sealing member **62** to be pushed through the opening and also permit the liquid to flow through the opening **60** in the distal direction, through the cavity portion **55** in the needle hub and out through the needle. This position is shown in Figure 4.

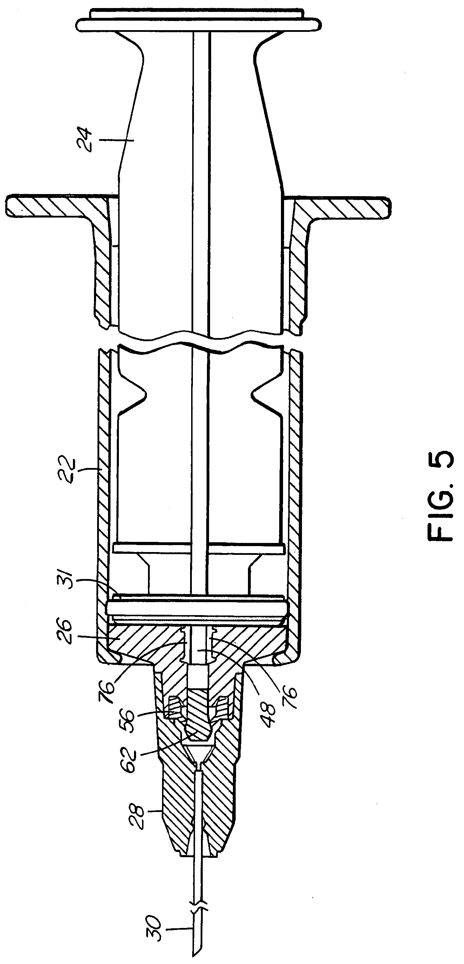

From this point, movement of the moveable sealing member **62** in the proximal direction is stopped by the engagement of the sealing portion **58** of the ring **56** with the head **66** of the moveable sealing member. The sealing portion **58,** and more specifically its sealing surface **90** around the opening **60,** seals against the head **66,** stopping any flow of liquid in the proximal direction. The sealing member and sealing ring thus act as a one-way valve in the syringe, a mechanism which does not require that the plunger be in the fully inserted position in order to prevent refilling. As the plunger is pushed farther to complete the injection, emptying the barrel, the locking pins **76** enter the enlarged portion **48** of the channel **42** of the needle connector. The ribs **80** lock into the grooves **50** of the channel **42.** The needle connector **26** is thus securely attached to the plunger **24,** as illustrated in Figure 5.

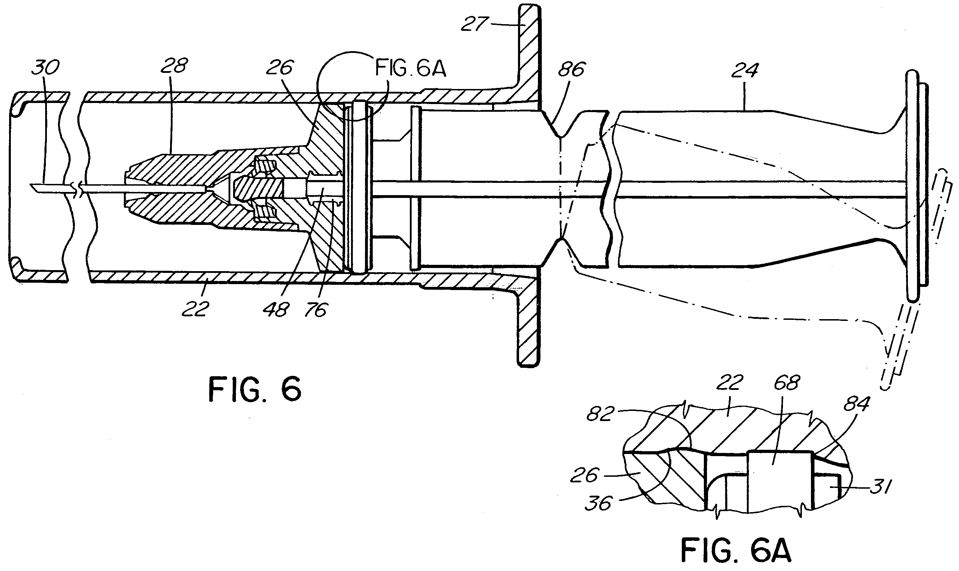

The plunger **24** is then retracted. As the plunger is moved in the proximal direction, the snap-fit attachment of the needle connector to the barrel wall **23** at the distal end of the barrel is overcome, permitting the needle connector to be withdrawn into the barrel bore, bringing with it the attached needle hub, and thus withdrawing the needle into the bore. This position is shown in Figure 6. The plunger is withdrawn until the radially outer periphery **36** of the needle connector and the O-ring **68** engage the barrel circumferential groove **82** and circumferential lip **84** respectively, locking the plunger in place. The length of the needle is such that the needle is now in a fully retracted position within the barrel bore. The user then breaks off at the weakened point **86** the portion of the shaft extending beyond the proximal end of the barrel. The syringe may then be safely discarded.

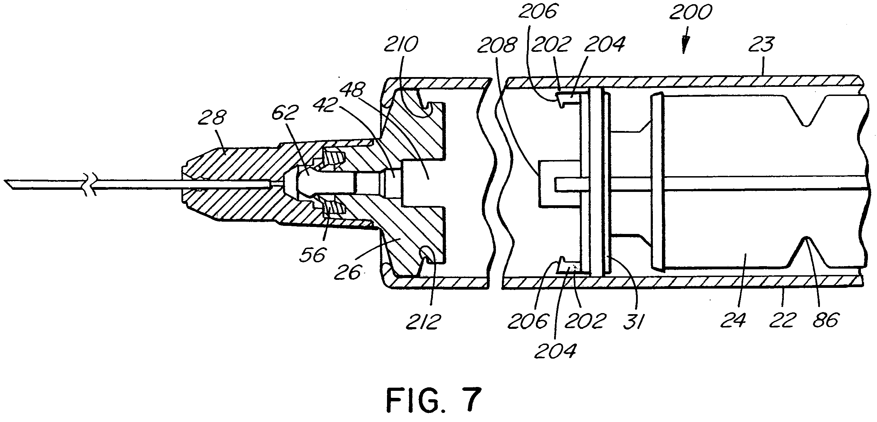

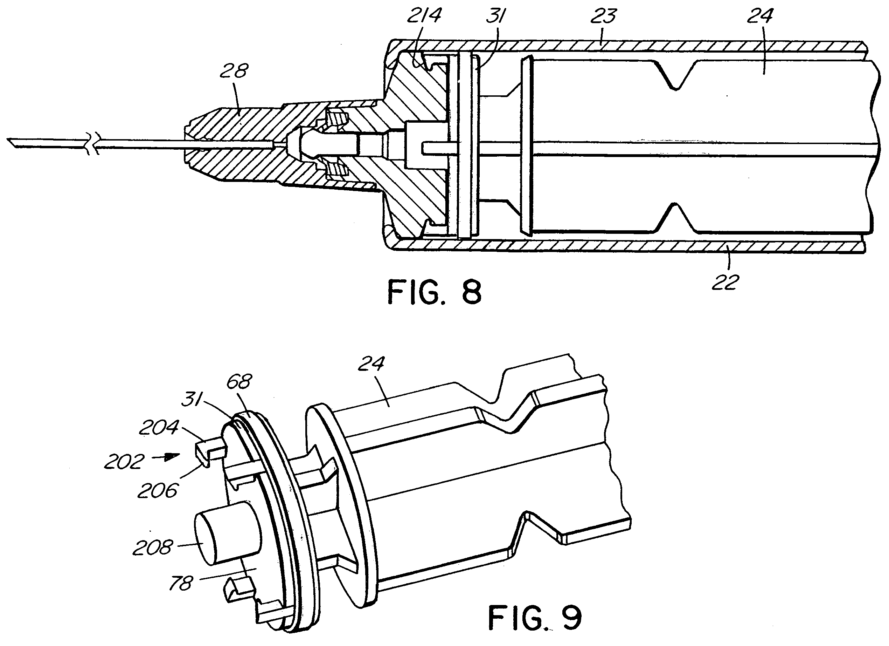

A second embodiment of the safety syringe is shown in Figures 7 to 9. The syringe **200** is essentially the same as the syringe **20** described above, except for the mechanism for locking together the needle connector and the plunger, and a structure for minimizing the volume of liquid left in the syringe after use.

The distal face **78** of the plunger has four locking pins **202** disposed at its periphery and extending distally and equally spaced apart. Each locking pin **202** has a leg **204** and an inwardly-extending hook portion **206.** A projecting member, cylindrical post **208,** extends distally from the center of the face **78.** The needle connector **26** has four peripheral recesses **210,** sized and spaced to be engaged by respective locking pins **202,** with a lip **212** for engagement by the hook **206** of the pin **202.** The channel **42** of the needle connector has an enlarged portion **48** sized to receive the post **208** of the plunger.

When the plunger is moved distally to engage the needle connector, the sloping surface **214** on the top of the resilient pins **202** causes the pins to be bent outwardly, into the space between the pins and the barrel wall **23.** The pins resile inwardly as the hooks **206** fit around the lip **212** of the needle connector recesses. The post **208** of the plunger then occupies the volume of the channel enlargement **48,** thus minimizing the volume of liquid left in the needle connector after use.

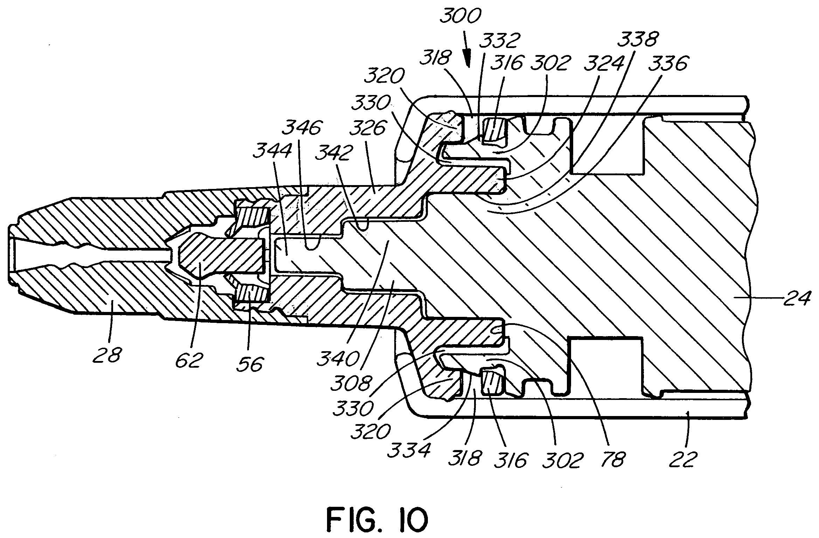

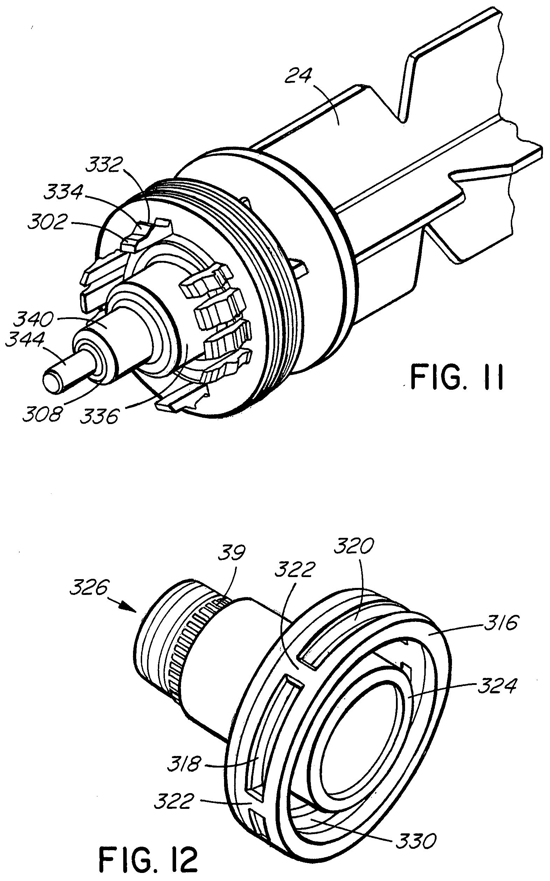

An embodiment of the safety syringe according to the invention is shown in Figures 10 to 12. The syringe **300** is essentially the same as the syringe **20** described above, except for the mechanism for locking together the needle connector and the plunger, and a structure for minimizing the volume of liquid left in the syringe after use.

The distal face **78** of the plunger has ten locking pins **302** disposed radially inwardly of its periphery and extending distally. The locking pins are arranged in four sets around a distally-extending post **308.** Two opposed sets have four locking pins each. The other two opposed sets have a single locking pin each. Each locking pin **302** has an outwardly-projecting hook **332** subtended by a sloping surface **334** on the outward surface of the pin.

The needle connector **326** has an annular member **316** that is spaced by a radially-extending slot **318** from a flange **320.** The annular member **316** is joined to the flange **320** by four connectors **322,** dividing the slot **318** into four segments.

The needle connector **326** has a cylindrical wall **324** extending distally from its proximal end, the wall **324** defining a first enlarged portion **338** of the channel **42** of the needle connector. The wall **324** is radially spaced from the annular member **316** and the flange **320** by an axially-extending annular groove **330,** which is sized to receive the locking pins **302.**

The post **308** on the distal face of the piston has three sections which are successively smaller in the distal direction and are shaped to be received within respective sections of the channel **42** of the needle connector. The first section **336,** being the largest in diameter, fits within the first section **338** of the channel; the second section **340** of the post **308** fits within the second section **342** of the channel; and the third section **344** of the post, being the smallest in diameter, fits within the smallest section **346** of the channel.

When the plunger is moved distally to engage the needle connector, the sloping outer surface **334** of the locking pins slides over the inner face of the annular member **316,** causing the locking pins to bend inwardly. As the hooks fit into the slot **318** the pins resile outwardly so that the hooks engage the annular member **316.** The spacing of the locking pins **302** and of the connectors **322** is such that, although some pins may align with a connector and not engage in the slot **318,** at least four pins will engage in the slot, irrespective of the orientation of the plunger, forming a sufficiently secure attachment between the plunger and the needle connector for retraction of the needle.

In the locked position, the post **308** of the plunger occupies the volume of the three sections **338, 342, 346** of the channel **42,** minimizing the volume of liquid left in the needle connector after use.

The components of the syringe may be made of any suitable materials. Such materials include polystyrene, polypropylene or polyethylene for the needle connector and the needle hub, acrylonitrile butadiene styrene (ABS) for the moveable sealing member, thermoplastic elastomer (TPE) for the sealing ring, and polypropylene for the barrel and plunger.

As will be apparent to those skilled in the art in the light of the foregoing disclosure, many alterations and modifications are possible in the practice of this invention without departing from the scope thereof.

### List of Reference Numerals in the Drawings

- 20: syringe
- 22: barrel
- 23: barrel wall
- 24: plunger
- 25: bore of barrel
- 26: needle connector
- 27: flange of plunger
- 28: needle hub
- 29: shaft of plunger
- 30: needle
- 31: piston
- 32: first portion of needle connector
- 33: second portion of needle connector
- 34: opening in distal end of barrel
- 35: thumb rest of plunger
- 36: outer periphery of needle connector
- 38: inner surface of barrel wall
- 39: ribs on needle connector
- 40: assembly of needle connector and needle hub
- 41: grooves on needle hub
- 42: channel in needle connector
- 43: longitudinal grooves along channel 42
- 44: proximal side of needle connector
- 46: distal side of needle connector
- 48: enlargement in channel 42
- 50: radial grooves in channel 42
- 52: cavity in assembly
- 53: cavity portion for sealing ring
- 54: annular base of sealing ring
- 55: cavity portion in needle hub
- 56: sealing ring
- 57: channels in cavity portion 55
- 58: sealing portion of sealing ring
- 60: opening in sealing portion of sealing ring
- 62: moveable sealing member
- 64: shaft of moveable sealing member
- 66: head of moveable sealing member
- 68: O-ring
- 70: opening at distal end of channel 42
- 76: locking pins
- 78: distal face of plunger
- 80: ribs on locking pins
- 82: groove in barrel wall
- 84: lip in barrel wall
- 86: frangible portion of plunger shaft
- 90: sealing surface of sealing ring
- 200: syringe (second embodiment)
- 202: locking pins
- 204: leg of locking pin
- 206: hook of locking pin
- 208: post on distal face of piston
- 210: peripheral recesses of needle connector
- 212: lip of peripheral recess
- 214: sloping surface of locking pin
- 300: syringe (third embodiment)
- 302: locking pins
- 308: post on distal face of piston
- 316: annular member of needle connector
- 318: radial slot in needle connector
- 320: flange
- 322: connectors
- 324: cylindrical wall of needle connector
- 326: needle connector
- 330: annular groove in needle connector
- 332: hook of locking pin
- 334: sloping outer surface of locking pin
- 336: first section of post 308
- 338: first section of channel 42
- 340: second section of post
- 342: second section of channel
- 344: third section of post
- 346: third section of channel

## Claims

1. A single use hypodermic syringe (300), comprising:
a barrel (22) having a barrel wall (23) defining a bore (25);
a plunger (24) slidably moveable within the barrel;
a needle connector (326) at the distal end of the barrel having a first portion (32) within the barrel bore and a second portion (33) extending from an opening (34) in the distal end of the barrel, the needle connector being in sealing, releasable engagement with the barrel wall;
a needle hub (28) affixed to the needle connector and having a hypodermic needle (30) fixed thereto;
the needle connector and the needle hub forming an assembly (40), the assembly having a cavity (52) therein, the needle and the barrel bore being in fluid communication through the cavity;
an elastically-deformable sealing ring (56) in the cavity having an opening (60) therein;
a sealing member (62) in the cavity having a head (66) and a shaft (64), the sealing member being moveable in the cavity, by means of fluid movement in the cavity, from a first position in which the head is on the proximal side of the ring to a second position in which the head is on the distal side of the ring;
the ring being configured to stop movement of the sealing member from the second position to the first position and to form a seal with the sealing member, when the sealing member is in the second position, against a flow of fluid in the proximal direction;
locking means for locking together the plunger and the needle connector;
**characterized in that**
the needle being retractable into the barrel bore, when the plunger and the needle connector are locked together, by movement of the plunger in the proximal direction[[.]];
and that the locking means comprises a plurality of latching members (302) on the plunger (24) at its distal face (78), spaced from the radially outer periphery thereof, and mating attachment means (316, 318, 330) for latching members in the needle connector (326);
wherein the latching members (302) have radially outwardly-extending projections (332);
wherein the projections (332) are subtended by a sloping surface (334) on the outward surface of the latching members (302) for engagement with an annular member (316) of the needle connector; and
wherein the mating attachment means comprises an axially-extending annular groove (330) in the needle connector to receive the latching members (302) and a radially-extending slot (318) in the needle connector to receive the projections (332) on the latching member.

2. A syringe according to claim 1, wherein the radially-extending slot (318) in the needle connector (326) comprises a plurality of segments.

3. A syringe according to claim 1, wherein the mating engagement means in the needle connector (326) further comprises an annular member (316) spaced by the radially-extending slot (318) from a flange (320), the annular member (316) being joined to the flange (320) by connectors (322).

4. A syringe according to claim 3, wherein the connectors (322) divide the radially-extending slot (318) into segments.

5. A syringe according to claim 4, wherein there are four connectors (322) and four segments of the radially-extending slot (318).

6. A syringe according to claim 3, wherein the projections (332) of the latching members (302) fit into the radially-extending slot (318) to engage the annular member (316) of the needle connector (326).

7. A syringe according to claim 6, wherein the projections (332) of at least four latching members (302) fit into the radially-extending slot (318) to engage the annular member (316) of the needle connector (326).

8. A syringe according to claim 3, wherein the needle connector (326) has a cylindrical wall (324) extending distally from its proximal end, the wall (324) being radially spaced from the annular member (316) and the flange (320) of the needle connector (326) by the axially-extending annular groove (330).

## Patentansprüche

1. Hypodermische Einwegspritze (300), umfassend:
einen Körper (22), aufweisend eine Körperwand (23), definierend eine Bohrung (25);
einen Kolben (24), der verschiebbar im Körper beweglich ist;
einen Nadelanschluss (326) am distalen Ende des Körpers, aufweisend einen ersten Abschnitt (32) in der Körperbohrung und einen zweiten Abschnitt (33), der sich von einer Öffnung (34) im distalen Ende des Körpers erstreckt, wobei der Nadelanschluss in abdichtendem, lösbarem Eingriff mit der Körperwand ist;
einen Nadelansatz (28), der am Nadelanschluss befestigt ist und eine hypodermische Nadel (30), die daran fixiert ist, aufweist;
wobei der Nadelanschluss und der Nadelansatz eine Einheit (40) bilden, wobei die Einheit einen Hohlraum (52) aufweist und die Nadel und die Körperbohrung durch den Hohlraum in Fluidkommunikation stehen;
einen elastisch verformbaren Dichtungsring (56) im Hohlraum, aufweisend eine Öffnung (60);
ein Dichtungselement (62) im Hohlraum, aufweisend einen Kopf (66) und eine Welle (64), wobei das Dichtungselement im Hohlraum mittels Fluidströmung im Hohlraum von einer ersten Position, in der der Kopf sich auf der proximalen Seite des Rings befindet, in eine zweite Position, in der sich der Kopf auf der distalen Seite des Rings befindet, bewegbar ist;
wobei der Ring ausgelegt ist, um die Bewegung des Dichtungselements von der zweiten Position in die erste Position zu stoppen und eine Dichtung mit dem Dichtungselement zu bilden, wenn sich das Dichtungselement in der zweiten Position befindet, und zwar gegen eine Fluidströmung in die proximale Richtung;
Spannmittel, um den Kolben und den Nadelanschluss zusammen festzuspannen;
**dadurch gekennzeichnet, dass** die Nadel in die Körperbohrung zurückgezogen werden kann, wenn der Kolben und der Nadelanschluss zusammen festgespannt werden, und zwar durch die Bewegung des Kolbens in die proximale Richtung,
und dadurch, dass die Spannmittel eine Vielzahl an Verriegelungselementen (302) am Kolben (24) an dessen distaler Fläche (78) umfassen, beabstandet von dessen radial äußerer Begrenzungsfläche, und Anschlussbefestigungsmittel (316, 318, 330) für Verriegelungselemente im Nadelanschluss (326);
wobei die Verriegelungselemente (302) sich radial nach außen erstreckende Vorsprünge (332) aufweisen;
wobei die Vorsprünge (332) von einer geneigten Oberfläche (334) auf der nach außen gerichteten Oberfläche der Verriegelungselemente (302) unterspannt sind, um mit einem ringförmigen Element (316) des Nadelanschlusses in Eingriff zu gelangen, und
wobei die Anschlussbefestigungsmittel eine sich axial erstreckende ringförmige Nut (330) im Nadelanschluss umfassen, um die Verriegelungselemente (302) aufzunehmen, und eine sich radial erstreckende Aussparung (318) im Nadelanschluss, um die Vorsprünge (332) der Verriegelungselemente aufzunehmen.

2. Spritze nach Anspruch 1, wobei die sich radial erstreckende Aussparung (318) im Nadelanschuss (326) eine Vielzahl an Segmenten umfasst.

3. Spritze nach Anspruch 1, wobei die Anschlusseingriffsmittel im Nadelanschluss (326) zudem ein ringförmiges Element (316) umfassen, das durch die sich radial erstreckende Aussparung (318) von einem Flansch (320) beabstandet ist, wobei das ringförmige Element (316) durch Konnektoren (322) mit dem Flansch (320) verbunden ist.

4. Spritze nach Anspruch 3, wobei die Konnektoren (322) die sich radial erstreckende Aussparung (318) in Segmente teilen.

5. Spritze nach Anspruch 4, wobei vier Konnektoren (322) und vier Segmente der sich radial erstreckenden Aussparung (318) vorhanden sind.

6. Spritze nach Anspruch 3, wobei die Vorsprünge (332) der Verriegelungselemente (302) in die sich radial erstreckende Aussparung (318) eingepasst werden, um mit dem ringförmigen Element (316) des Nadelanschlusses (326) in Eingriff zu gelangen.

7. Spritze nach Anspruch 6, wobei die Vorsprünge (332) von mindestens vier Verriegelungselementen (302) in die sich radial erstreckende Aussparung (318) eingepasst werden, um mit dem ringförmigen Element (316) des Nadelanschlusses (326) in Eingriff zu gelangen.

8. Spritze nach Anspruch 3, wobei der Nadelanschluss (326) eine zylindrische Wand (324) aufweist, die sich distal von ihrem proximalen Ende erstreckt, wobei die Wand (324) radial vom ringförmigen Element (316) und vom Flansch (320) des Nadelanschlusses (326) durch die sich axial erstreckende ringförmige Nut (330) beabstandet ist.

## Revendications

1. Seringue hypodermique jetable (300) comprenant :
un corps de seringue (22) ayant une cloison de corps de seringue (23) définissant un alésage (25) ;
un piston (24) pouvant se déplacer en coulissant à l'intérieur du corps de seringue ;
un raccord d'aiguille (326) situé au niveau de l'extrémité distale du corps de seringue ayant une première partie (32) à l'intérieur de l'alésage du corps de seringue et une seconde partie (33) se prolongeant à partir d'une ouverture (34) dans l'extrémité distale du corps de seringue, le raccord d'aiguille étant en prise de façon amovible et par scellement avec la cloison du corps de seringue ;
un pavillon d'aiguille (28) fixé au raccord d'aiguille et ayant une aiguille hypodermique (30) fixée à celui-ci ;
le raccord d'aiguille et le pavillon d'aiguille formant un ensemble (40), l'ensemble disposant d'une cavité (52) en son sein, l'aiguille et l'alésage du corps de seringue étant en communication fluidique par la cavité ;
un joint d'étanchéité déformable élastiquement (56) dans la cavité ayant une ouverture (60) en son sein ;
un élément d'étanchéité (62) dans la cavité ayant une tête (66) et une tige (64), l'élément d'étanchéité étant mobile dans la cavité, au moyen d'un déplacement fluidique dans la cavité, d'une première position dans laquelle la tête se situe au niveau du côté proximal du joint, à une seconde position dans laquelle la tête se situe au niveau du côté distale du joint ;
le joint étant configuré pour arrêter le déplacement de l'élément d'étanchéité de la seconde position à la première position et pour former un scellement avec l'élément d'étanchéité lorsque l'élément d'étanchéité se trouve dans la seconde position contre un écoulement de fluide dans la direction proximale ;
un moyen de blocage destiné à bloquer ensemble le piston et le raccord d'aiguille ;
**caractérisée en ce que** l'aiguille est rétractable dans l'alésage du corps de seringue, lorsque le piston et le raccord d'aiguille sont bloqués ensemble, par le déplacement du piston dans la direction proximale,
et **en ce que** le moyen de blocage comprend une pluralité d'éléments de verrouillage (302) sur le piston (24) au niveau de sa face distale (78), espacés de sa périphérie radialement extérieure, et des moyens de fixation d'accouplement (316, 318, 330) pour éléments de verrouillage dans le raccord d'aiguille (326) ;
dans laquelle les éléments de verrouillage (302) possèdent des saillies (332) se prolongeant radialement extérieurement ;
dans laquelle les saillies (332) sont sous-tendues par une surface inclinée (334) sur la surface extérieure des éléments de verrouillage (302) pour se mettre en prise avec un élément annulaire (316) du raccord d'aiguille ; et
dans laquelle le moyen de fixation d'accouplement comprend une rainure annulaire (330) se prolongeant axialement dans le raccord d'aiguille pour recevoir les éléments de verrouillage (302) et une fente (318) se prolongeant radialement dans le raccord d'aiguille pour recevoir les saillies (332) sur l'élément de verrouillage.

2. Seringue selon la revendication 1, dans laquelle la fente (318) se prolongeant radialement dans le raccord d'aiguille (326) comprend une pluralité de segments.

3. Seringue selon la revendication 1, dans laquelle le moyen de mise en prise d'accouplement dans le raccord d'aiguille (326) comprend de plus un élément annulaire (316) espacé par la fente (318) se prolongeant radialement à partir d'une bride (320), l'élément annulaire (316) étant joint à la bride (320) par des raccords de jonction (322).

4. Seringue selon la revendication 3, dans laquelle les raccords de jonction (322) divisent la fente (318) se prolongeant radialement en segments.

5. Seringue selon la revendication 4, dans laquelle se trouvent quatre raccords de jonction (322) et quatre segments de la fente (318) se prolongeant radialement.

6. Seringue selon la revendication 3, dans laquelle les saillies (332) des éléments de verrouillage (302) se logent dans la fente (318) se prolongeant radialement pour se mettre en prise avec l'élément annulaire (316) du raccord d'aiguille (326).

7. Seringue selon la revendication 6, dans laquelle les saillies (332) d'au moins quatre éléments de verrouillage (302) se logent dans la fente (318) se prolongeant radialement pour se mettre en prise avec l'élément annulaire (316) du raccord d'aiguille (326).

8. Seringue selon la revendication 3, dans laquelle le raccord d'aiguille (326) comporte une cloison cylindrique (324) se prolongeant de façon distale de son extrémité proximale, la cloison (324) étant espacée radialement de l'élément annulaire (316) et de la bride (320) du raccord d'aiguille (326) par la rainure annulaire (330) se prolongeant axialement.
